# NEW EUROPEAN PATENT SPECIFICATION

(11) **EP 1 009 385 B2**
(45) Date of publication and mention of the opposition decision: **03.01.2007**
(45) Mention of the grant of the patent: 09.07.2003
(21) Application number: 99948541.0
(22) Date of filing: 30.04.1999
(51) Int. Cl.: A61K 9/14, A61K 31/575

(54) **CHOLESTEROL LEVEL LOWERING COMPOSITION**
ZUSAMMENSETZUNG ZUR SENKUNG DES CHOLESTERINSPIEGELS
COMPOSITION HYPOCHOLESTEROLEMIANTE

(30) Priority: 30.04.1998 SE 9801536
(43) Date of publication of application: 21.06.2000
(73) Proprietor: Triple Crown AB, 182 38 Danderyd (SE)
(72) Inventor: SJÖBERG, Kjell, S-182 38 Danderyd (SE)
(86) International application number: PCT/SE1999/000721
(87) International publication number: WO 1999/056729

(56) References cited:
- EP-A- 0 898 896
- EP-A- 0 947 197
- EP-A- 0 990 391
- EP-A- 1 197 153
- EP-A1- 0 357 967
- WO-A-94/20072
- WO-A-99/43218
- DE-A1- 4 038 385
- GB-A- 1 365 661
- JP-A- 6 329 588
- US-A- 4 218 334
- Pollak, OJ Pharmac. Ther. 31:177-208 (1985)
- Nutrafoods abstract
- DATABASE WPI Week 8728, Derwent Publications Ltd., London, GB; AN 87-196297 NISSHIN OIL MILLS LTD: 'Instant powder compsns with improved dispersibility - contg. lecithin and sterol (deriv.)' & JP 62 126 966 A 09 June 1987

## Description

### Technical field.

The present application describes a composition containing as cholesterol lowering component, β-sitosterol and/or β-sitostanol, food containing such a composition, and a method to prepare the composition.

### Background of the invention.

A daily intake of some compounds similar to cholesterol has been shown to have a cholesterol lowering effect. Specifically this is true for β-sitosterol and the hydrogenated form β-sitostanol (1-5).

Under β-sitosterol is also understood mixtures containing β-sitosterol, β-sitostanol and campesterol isolated from for instance soy or tall oil. Under β-sitostanol is also understood fully or partly hydrogenated β-sitosterol as above.

It is known that sterols and stanols are compounds with a very low solubility. They also crystallise easily. Several researchers have pointed at the importance that β-sitosterol and β-sitostanol must be adminstered in a formulation giving the optimal cholesterol lowering effect in the body. In a crystalline form even after efficient micronisation and/or in suspension the effect is lower than for solutions or emulsions (6-15). Hitherto described solutions and emulsions, however, have the disadvantage being too dilute to allow a simple in-take in doses nescessary of about 1.5 g/day (16-18).

GB-A-1,365,661, relates to glycosides of sterols and in particular to a method for preparing a composition for medicinal or food purposes. The method encompasses the steps of dissolving β-sitosteryl-β-D-glucoside in a liquid medium and adding the resultant coposition in a monomolecularly dispersed state to a carrier selected from absorbent solids and emulsions. GB-A-1,365,661 does not relate to unmodified sterols as the present invention does.

It is thus not taught that the unmodified β-sitosterol is present in monomolecular form, but that a glucoside is present, which is contrary to the present invention.

EP-A-0 357 967 concerns β-sitosterol and/or β-sitostanol as such for treating cholesteroiaemia with a content of phytosterols or their derivatives with improved water solubility. These compounds are present in an organic phase. However, this organic phase is not distributed, immobilised, and stabilized in a matrix. Besides the solubility of sterols in polyethylene glycols is about 2.5% at 20°C, which is far too low for achieving concentrations according to the present invention. The product thereof will result in a daily intake of 60 capsules of 1 g to reach the recommended dose.

DE-A-4,038,385 is concerned with microemulsions of sitosterol glucosides. Such microemulsions are distinguished by the fact that the concentrations of the participating components can vary only within very narrow, limited ranges. The microemulsions disclosed in the document are not edible and are toxic due to the presence of isopropanol.

JP-A-62 126 966 is concerned with the problem of providing a composition of an instant powder (e. g., milk powder) with improved solubility and/or dispersability toward cold water. In order to improve the solubility, lecithin in combination with sterols or sterol derived compounds are added to the instant powder. The concentration of sterol is far too low for providing an cholesterol lowering effect in daily doses. Lecithin is used as a conventional surface active ingredient and not as a solvent. Accordingly the solution of JP-A-62 126 966 is not concerned with the same problem as the present invention.

WO 98/43218, which is a document according to Article 54(3)(4) EPC refers to a method for producing a fatty blend of plant sterol in a partly dissolved and for microcrystalline form.

The solubility in fat of sterols and stanols, both being alcohols, can be increased considerably by esterification with fatty acids. These esters are hydrolysed in the stomach and sterols and stanols are liberated as the initial alcohols in a concentration low enough not to allow recrystallisation. The cholesterol lowering effect in the gut is thus improved (16-20).

We have now shown that it is not necessary to esterify sterols and stanols to be able to distribute them in a sufficiently high concentration in a monomolecular, low associated or "cluster form" to reach necessary daily doses.

### Description of the Invention

The present invention is as set out in the claims.

In the present invention we show how the sterols and stanols by simple methods can be stabilised in monomolecular, low associated or "cluster" form by distributing and immobilising a solution of high concentration or a melt of sterols and/or stanols in a matrix.
The present invention relates to a process for manufacturing a composition containing a cholesterol lowering component containing beta-sitosterol and/or beta-sltostanol in a monomolecular, low associated or cluster form, comprising
- melting or dissolving the beta-sitosterol and/or beta-sitostanol in an organic phase selected from mono; and diglycerides or mixtures thereof, at a temperature of 60 to 160°C,
- and - before the melt or solution crystallizes or associates in another way - distributing it in a matrix so that the mentioned component is stabilized mainly in mono molecular or low associated or cluster form, wherein the matrix is based on a solid or highly viscous material selected from gelatine, casein, pectin, agar, starch, starch syrup, ethyl hydroxyethyl cellulose, stearic acid, chocolate mass or a mixture of two or more of these.

The compositions as obtained by the claimed process can also be mixed into different food e.g. chocolate, dough/bread, jelly, mashed potatoes, butter/margarine, youghurt and others or be encapsulated or mixed into tablets.

The special characteristics of the present innovation are also shown by the enclosed claims.

The present innovation is described below by not limiting examples, If not otherwise stated the given values are in weight or weight %.

### Example 1.

30 g of β-sitostanol were dissolved in 70 g of a monoglyceride such as Dimodan RT at 90° C on a hot water bath and stirred till the stanol was completely dissolved. The 30% solution can be used directly or stored as a prefabricate after cooling as a homogenous mass. 20 g of the solution obtained were slowly added under intensive stirring to 80 g of a 35% solution of gelatin and agar 10:1 , starch syrop, sugar and aroma in water placed on a hot water bath at 65 °C.
The liquid highly viscous composition was immediately cast in small forms, where it solidified as a gel containing β-sitostanol in a stabilised monomolecular or low associated form.
The composition can be used as such or be mixed into food and/or be foamed, encapsulated or made into tablets.

### Example 2.

10 g of β-sitostanol were dissolved in 10 g of Dimodan RT at 120°C on an oil bath. The solution was carefully under intensives stirring added to 80 g of a 30% solution of gelatin in water, also containing sugar and aroma, placed on a water bath of 85 °C. The homogenous stabilised composition was then treated as in example 1.

### Reference Example 3.

10 g of β-sitosterol were dissolved in 10 g of rapeseed oil at 120 °C on an oil bath. The solution was carefully under intensive continous stirring added to 30 g of a 30% solution of gelatin in water, containing sugar and aroma, placed on a hot water bath of 95 °C.
The homogenous stabilised composition was then treated as in example 1.

### Reference Example 4.

10 g of β-sitostanol were dissolved in 10 g of rapeseed oil at at 130 °C on an oil bath. The solution was carefully added under intensive continous stirring to 50 g of a 30% solution of gelatin in water, containing sugar and aroma, placed on a hot oil bath of 110°C.
The homogenised stabilised composition was then treated as in example 1.

### Example 5.

10 g of β-sitostanol were dissolved in 10 g of Dimodan RT at 120 °C on an oil bath. In another flask 80 g of chocolatemass were melted on the same bath. The 50% solution of β-sitostanol was slowly added under continous stirring to the chocolate. The composition was directly cast in forms, where it solidified and could be used as such or mixed into food or tableted, encapsulated or foamed.

### Reference Example 6.

10 g of β-sitostanol were dissolved in 10 g of rapeseed oil at 130 °C on an oil bath. In another flask 40 g of chocolatemass were melted at 110 °C on an oil bath. The 50% solution of β-sitostanol was added under continous stirring to the chocolate.
The composition was directly cast in forms where it solidified and can be used as such or mixed inte food or be tabletted, encapsulated or foamed.

### Example 7.

2.5 g of β-sitosterol were dissolved in 2.5 g of Dimodan ML at 80 °C. The solution was added to 1 litre of a 3% solution of sodium caseinate at 60-80 °C under strong stirring with a Turrax. After 5 minutes the emulsion was cooled to 20 °C and can be used as a beverage. The emulsion can be kept in a fridge during at least 3 days. The same result is obtained using milk.

### Example 8.

12.5 g of β-sitosterol were dissolved in 25 g of Dimodan ML at 60 °C. The solution was added to 80 g of melted margarine and mixed with 125 g of flour, 175 g of oatflakes, 2.5 g of baking powder and 80 g of sugar to a dough. The mixture was baked into 25 cakes of 18-20 g each containing 0.5 g of β-sitosterol.

### REFERENSES

1 Pollak, O.J., Reduction of blood cholesterol in man. Cirkulation, 7, 702-706, (1953)
2 Grundy, S.M., Ahrens, E. H. Jr., and Davignon, J., The interaction of cholesterol absorption and cholesterol synthesis in man. J. Lipid Res., 10, 304, (1969).
3 Farguhar, J.W..and Sokolow, M., Responce of serum lipids and lipoproteins of man to beta-sitosterol and sanflower oil - Along term study, Cirkulation, 17, 890,(1956).
4 Oster, P., Schlierf, G., Heuck, C.C., Greten, H., Gundert-Remy, U., Haase, W. Klose, G., Nothelfer, A.,Raetzer, H., Schellenberg, B. und Schmidt-Gauk, H., Sitosterin beifamiliären Hyperlipoproteinammie Tyo II. Eine randomisierte gekreuzte Doppelblindstudie, Dtsch, Med. Wschr., 101, 1308-1311, (1976).
5 Grundy, S.M., Mok, H.Y.I., Effekts of low dose phytosterols on cholesterol absorption in man, "Lipoprotein metabolism".,p114-118 ,Ed greten, H., Berlin, Heidelberg, New York,Springer-Verlag, (1976).
6 Miettinen,T.A., Siurala, M., Bile salts, sterols, sterol esters glycerides and fatty acids in micellar and oil phases of inerstinal contents during fat digestion in man, Z. Klin. Chem. Biochem., 9, 47-52, (1979)
7 Kudchodkar,B.J, Horlick, L., Sodhi, H,S. Effekts of plant sterols on cholesterol metabolism in man, Atherosclerosis, 23, 239 ,(1976).
8 Hassan, A.s., Rampone, A. J., Intestinal absorption and lymhatic transport of cholesterol and β-sitostanol in the rat, J. Lipid Res., 20, 646-653, (1979).
9 Heinemann, T., Kullak-Ublick, G.-K., Pietruck, B., von Bergman, K., Mechanisms of action of plants sterols on inhibition of cholesterol absorption, Eur. J. Clin. Pharmacol., 40 Suppl., 50-63,(1991).
10 Ikeda, I., Tanaka, K., Sugano, M., Vahouny, G. V., Gallo, I L,. Inhibition of cholesterol absorption in rats by plant sterols, J. Lipid Res., 29, 1573-1582, (1988)
11 Ikeda, I., Tanaka, K., Sugano, M., Vahouny, G. V., Gallo, I L,. Discrimination between cholesterol and sitosterol for absorption in rats, J. Lipid Res., 29,1583-1592, (1988)
12 Ikeda, I., Tanabe, Y and Sugano, M., Effects of sitosterol och sitotanol on micellar solubility of cholesterol, J. Nutr. Sci. Vitaminol., 35, 361-369, (1989).
13 Ikeda, I., Sugano, M., Comparison of absorption and metabolism of beta-sitosterol in rats, Atherosclerosis, 30, 227, (1978)
14 Sugano, M., Marioka, H. and lkeda, I., A comparison of hypocholesterolemic activity of β-sitosterol och β-sitostanol in rats, J., Nutr., 107, 2011-2019,(1977).
15 Heinemann, T., Pietruck,B., Kullak-Ublick, G.-K., Pietruck, B., von Bergman, K.,Comparison of sitosterol and sitostanol on inhibition of intestinal cholesterol absorption,, Agents Action (Suppl), 26, 117-122, (1988).
16 Heinemann, T., Leiss, O., B., von Bergman, K., Effects of low-dose sitotanol onserum cholesterol in patients with hypercholesterolemia, Atherosclerosis, 61, 219-223, (1986).
17 Miettinen, T.A., Vanhanen,H., Dietary sitostanol related to absorbtion, syntesis and serum level of cholestol in different apolipoprotein e- phenotypes, Atherosclerosis, 105, 217-226, (1994).
18 Mattson, F.,H., Volpenstein, R.,A., Erickson, B., A., Effects of plant sterol esters on the absorption of dietary cholesterol, J. Nutr., 107, 1139-1146 (1977)
19 Mattson, F.,H., Grundy, S.M., Crouse, J.R., Optimizing the effekt of plant sterols on cholesterol absorptionin man, Am. J. Clin. Nutr., 35, 697-700, /1982).
20 A substance for lowering high cholesterol level in serum and method for preparing the same. Patent C07J 9/00, A61 K 31/575

## Claims

1. A process for manufacturing a composition containing a cholesterol lowering component containing beta-sitosterol and/or beta-sitostanol In a monomolecular, low associated or cluster form, comprising
- melting or dissolving the beta-sitosterol and/or beta-sitostanol in an organic phase selected from mono- and diglycerides or mixtures thereof, at a temperature of 60 to 160°C,
- and - before the melt or solution crystallizes or associates In another way - distributing it in a matrix so that the mentioned component is stabilized mainly in mono molecular or low associated or cluster form, wherein the matrix is based on a solid or highly viscous material selected from gelatine, casein, pectin, agar, starch, starch syrup, ethyl hydroxyethyl cellulose, stearic acid, chocolate mass or a mixture of two or more of these.

2. The process according to claim 1, wherein the temperature is in the interval of 60° to 150°C.

3. The process according to any one of the preceding claims, in which the organic phase contains a monoglyceride.

4. The process according to any one of the preceding claims, wherein said matrix is based on a 10-50 wt-% solution of gelatine in water, and, if chosen, taste and aroma compounds.

5. The process according to any one of the preceding claims, in which the matrix at room temperature is a solid solvent such as stearic acid.

6. The process according to any one of the preceding claims, In which the cholesterol lowering agent is 1-99 wt-% of the organic phase.

7. Composition obtainable by a process according to any one of the preceding claims.

8. Food containing a composition as in claim 7, in an amount to ensure a cholesterol lowering effect.

9. Food as in claim 8 being butter, butter substitute, margarine, chocolate, jelly, bread, mashed potatoes, yogurt, soup.

10. Food as in claims 8-9, in which mentioned composition is included in the food in up to 60 wt-%.

11. Capsule, tablet, or foam containing a composition as in claim 7.

## Patentansprüche

1. Verfahren für die Herstellung einer Zusammensetzung mit einer cholesterinsenkenden Komponente, welche Beta-Sitosterin und/oder Beta-Sitostanol in einer monomolekularen, niedrigassoziierten oder Clusterform enthält, umfassend
- das Schmelzen oder Auflösen des Beta-Sitosterin und/oder des Beta-Sitostanol in einer organischen Phase, ausgewählt aus Mono- und Diglyceriden oder Mischungen derselben, bei einer Temperatur von 60 bis 160° C,
- und - ehe die Schmelze oder die Lösung kristallisiert oder in einer anderen Weise assoziiert - Verteilen derselben in einer Matrix dergestalt, dass die erwähnte Komponente stabilisiert wird hauptsächlich in monomolekularer oder niedrigassoziierter oder in Clusterform, wobei
die Matrix auf einem festen oder hochviskosen Material beruht, ausgewählt aus Gelatine, Casein, Pektin, Agar, Stärke, Stärkesirup, Ethylhydroxyethylcellulose, Stearinsäure, Schokoladenmasse oder einer Mischung von zwei oder mehr derselben.

2. Verfahren nach Anspruch 1, **dadurch gekennzeichnet, dass** die Temperatur in einem Bereich von 60 bis 150°C liegt.

3. Verfahren nach einem der vorangehenden Ansprüche, **dadurch gekennzeichnet, dass** die organische Phase ein Monoglycyerid enthält.

4. Verfahren nach einem der vorangehenden Ansprüche, **dadurch gekennzeichnet, dass** die Matrix auf einer Lösung von Gelatine in Wasser von 10 - 50 Gewichtsprozent beruht, und, falls ausgewählt, von Geschmacks- und Aromaverbindungen.

5. Verfahren nach einem der vorangehenden Ansprüche, **dadurch gekennzeichnet, dass** die Matrix bei Raumtemperatur ein festes Lösungsmittel, wie zum Beispiel Stearinsäure, ist.

6. Verfahren nach einem der vorangehenden Ansprüche, **dadurch gekennzeichnet, dass** das cholesterinsenkende Mittel 1 - 99 Gewichtsprozent der organischen Phase darstellt.

7. Zusammensetzung, erzielbar durch ein Verfahren nach einem der vorangehenden Ansprüche.

8. Nahrungsmittel, die eine Zusammensetzung wie in Anspruch 7 enthalten, in einer Menge, die eine cholesterinsenkende Wirkung gewährleistet.

9. Nahrungsmittel wie in Anspruch 8, die Butter, Butterersatz, Margarine, Schokolade, Gelee, Brot, Kartoffelbrei, Joghurt, Suppe sind.

10. Nahrungsmittel wie in den Ansprüchen 8 und 9, **dadurch gekennzeichnet, dass** die erwähnte Zusammensetzung in den Nahrungsmitteln in einer Menge von bis zu 60 Gewichtsprozent vorliegt.

11. Kapsel, Tablette oder Schaum, welche eine Zusammensetzung wie in Anspruch 7 enthalten.

## Revendications

1. Un procédé de préparation d'une mixture comprenant une substance, diminuant le taux de cholestérol, constituée de bêta-sitostérol et/ou de bêta-sitostanol sous une forme monomoléculaire, faiblement associée ou de cluster, consistant :
- à faire fondre ou à dissoudre le bêta-sitostérol et/ou le bêta-silostanol dans une phase organique choisie parmi les monoglycérides, des diglycérides ou leurs mélanges, à une température comprise entre 60 et 160°C,
- et - avant que le fondant ou la solution cristallise ou s'associe d'une autre façon - le (ou la) répartir dans une matrice de telle façon que la substance mentionnée soit stabilisée principalement sous forme monomoléculaire ou faible associée ou de cluster,
selon lequel la matrice est constituée d'un solide ou d'un matériau fortement visqueux sélectionné parmi le/la gélatine, caséine, pectine, agar, amidon, sirop d'amidon, cellulose éthylhydroxyéthyle, acide stéarique, masse de chocolat ou un mélange de deux ou plus de deux-ci.

2. Le procédé selon la revendication 1, dans lequel la température se situe dans un intervalle compris entre 60 et 150°C.

3. Le procédé selon l'une quelconque des revendications précédentes, dans lequel la phase organique contient un monoglycéride.

4. Le procédé selon l'une quelconque des revendications précédentes, dans lequel la matrice est basée sur une solution aqueuse de gélatine à 10-50% en poids et, si choisi, avec des composés pour le goût et l'arôme.

5. Le procédé selon l'une quelconque des revendications précédentes, dans lequel la matrice à température ambiante est un solvant solide tel que l'acide stéarique.

6. Le procédé selon l'une des revendications précédentes, dans lequel l'agent diminuant le taux de cholestérol représente 1 à 99% en poids de la phase organique.

7. Mixture obtenue à partir d'un procédé selon l'une quelconque des revendications précédentes.

8. Aliment contenant une mixture telle que dans la revendication 7, dans une proportion assurant une diminution du cholestérol.

9. Aliment tel que dans la revendication 8, étant du beurre, des substitutifs du beurre, de la margarine, du chocolat, de la gelée, du pain, de la purée de pommes de terre, yoghourt ou soupe.

10. Aliment tel que les revendications 8-9, dans lequel le mélange mentionné est inclus dans l'aliment jusqu'à 60% en poids.

11. Capsule, tablette ou mousse contenant une composition telle que dans la revendication 7.
